# EUROPEAN PATENT APPLICATION

(11) **EP 1 329 219 A1**
(43) Date of publication of application: **23.07.2003**
(21) Application number: 01936944.6
(22) Date of filing: 11.06.2001
(51) Int. Cl.: A61K 31/685, A61K 31/683, A61P 43/00, A61P 35/00, A61P 35/02, A61P 37/06

(54) **APOPTOSIS INDUCERS, CASPASE CASCADE ACTIVATORS AND ANTICANCER AGENTS**

(30) Priority: 29.09.2000 JP 2000299631
(71) Applicant: Kimigafuchi Gakuen, Kumamoto-shi, Kumamoto 860-0082 (JP); Ueoka, Ryuichi, Kumamoto-shi, Kumamoto 862-0941 (JP)
(72) Inventor: UEOKA, Ryuichi, Kumamoto-Shi, Kumamoto 862-0941 (JP); MATSUMOTO, Yoko, Kumamoto-Shi, Kumamoto 862-0972 (JP)
(74) Representative: Horner, Martin Grenville
(86) International application number: JP0104903
(87) International publication number: WO02028399

(57) **Abstract**

The present invention provides an apoptosis inducer comprising, as active ingredients, a compound selected from the group consisting of di(alkylcarbonyl) phosphatidylcholine, di(alkylcarbonyl) phosphatidylalkanolamine,di(alkylcarbonyl) phosphatidylinositol and di(alkylcarbonyl) phosphatidylserine.

## Description

### Background Art

The present invention relates to a new apoptosis inducer, caspase cascade activator and anticancer agent.

The concept of apoptosis has been proposed in a report in 1972 by Kerr, Wyllie, and Currie. (Kerr J. F. R., Wyllie A. H., and Currie A. R., Br. J. Cancer, 26, 239∼257(1972)). Apoptosis is characterized by drastic configuration transformation of the shape such as shrinking of cells, condensation of chromatin, fragmentation of DNA into nucleosome pieces (approximately 180 bp). While necrosis (local death) is defined as spasmodic cell death due to physical influence from external stimulation, apoptosis is defined as controlled cell death by genes for maintaining an organism.

It is thought that the mechanism of apoptosis follows the three stages of induction, determination, and execution. The determination step playing a principal role with regard to the mechanism of apoptosis has two transmission pathways. One is a pathway through mitochondria, which is controlled by the Bcl-2 family. The other is a transmission pathway through self-decomposition by caspases and limited decomposition by the caspases downstream on the caspase cascade, so-called caspase cascade.

The molecular mechanism of the apoptosis has been investigated by Horvitz, Yuan, et al., using nematodes. They discovered a total of fourteen genes relating to apoptosis of cells, which constitutes a nematode, and found that ced-3 and ced-4 are important as apoptosis executing factors, and ced-9 is important as an apoptosis inhibiting factor. As a result of gene identification, it has been revealed that ced-3 has high homology to the human IL-1β converting enzyme (ICE) which is a kind of cysteine protease (Yuan J. Shaham S., Ledoux S., Ellis H. M., and Horvitz H. R., Cell, 75, 641∼652 (1993)). Moreover, it has been confirmed that apoptosis is induced from overexpression of ICE, so it has come to be thought that the ICE serves as an apoptosis executing factor in humans. However, there was little influence upon functions of apoptosis with regard to an ICE knockout mouse, which was reported subsequently. Also, subsequently, it has been found that decomposition of poly(ADP-ribose) polymerase, which is characteristic of apoptosis, is not brought about by ICE, but is brought about by the cysteine protease similar to ICE. Accordingly, it has been presumed that the apoptosis executing factor is not an ICE itself in humans, but rather, is a different enzyme, i.e., ICE-like enzyme. Thus, it has been thought that there are ICE-like enzymes other than ICE in humans.

Accordingly, identification of ICE-like enzymes have been attempted, after the report by Yuan et al. Then, it has been revealed that there are about ten kinds of ICE-like enzymes including ICE in humans. The identified ICE-like enzymes are Asp-specific-reactive cysteine protease, so the enzymes are uniformly referred to as "caspase". The enzymes are each named by adding the number in order of report to "caspase", for example, caspase-1 (ICE), ..., caspase-10, and so forth, and in general, the enzymes has been referred to as caspase family protease.

At present, the caspases are generally classified into three groups from substrate specificity. The group I mainly of the caspase-1 is thought to be related to generating and secreting of interleukin, the group II mainly of the caspase-3 is thought to be a principal executor in executing molecules, and the group III represented by caspase-8 and caspase-9 is thought to be situated upstream on the protein decomposing cascade of caspase, and to have functions for transmitting signals for death of cells by apoptosis.

The caspase family has an activation of cutting off the C-terminal side of Asp(D), due to the common active center sequence, and each of the caspase family has the substrate specificity, somewhat different one from another. In general, the caspase is generated as an inactive precursor. When the caspase is cut off into two sub-units by an activating enzyme, whereby an active caspase is configured. The cut-off site for activation of the precursor is the C-terminal side of the Asp residue within the caspase molecules, and is the same site as that of the caspase for recognizing the substrate. Accordingly, it is presumed that there is a mechanism wherein a caspase itself activates other caspases. In apoptosis induced by Fas, the phenomena is observed wherein activation of the caspase-3-like increases after activation of the caspase-1, and it is known that the caspase-3 is situated downstream on the caspase-1 (Enari M., Talanian R. V, Wong W. W., and Nagata S., Nature, 380, 723∼726(1996)). Thus, it is presumed that several kinds of caspases are sequentially activated in apoptosis executing processes, that is to say, the caspase cascade is configured. Also, the caspases decompose protein in cells, whereby the caspases play an important role in apoptosis-executing processes.

As described above, it is interesting that a number of caspases exist in humans, and it is thought that a complex caspase network is configured for maintaining an organism.

However, at present, a great part of the actual mechanism for activating the caspase cascade is still unknown.

As described above, it is expected that apoptosis and the caspase cascade, which play important roles in an organism, are also critical for developing pharmaceuticals, and therapeutic agents for cell-multiplication diseases such as cancer wherein the apoptosis-inducing mechanism does not function normally can be developed using the mechanism of apoptosis and the caspase cascade.

For example, Japanese Patent Un-examined Publication No. 2000-143532 discloses treatment of cell-multiplication diseases by activating the caspase cascade and inducing apoptosis using a drug having a ring-shaped RGD sequence as the active ingredient. However, apoptosis inducers and caspase cascade activators comprising as the active ingredient dimyristoylphosphatidylcholine have not been known.

On the other hand, several anticancer agents using lipid components have been known. In general, most anticancer agents comprising lipid components use the lipid component as carriers of drugs in the form of liposome or emulsion, and few anticancer agents have been known wherein the lipid component itself acts as an anticancer agent. Japanese Patent Publications describing effectiveness of lipid components on cancer will be described below. Japanese Patent Un-examined Publication No. 6-256181 discloses an cell-differentiation inducing agent comprising tocopherol as the active ingredient, which is effective in treatment and improvement of cancer. Japanese Patent Un-examined Publication No. 1-203330 discloses an anticancer agent comprising as the active ingredient docosahexaenoyl lysophosphatidylcholine. Japanese Patent Un-examined Publication No. 1-203322 discloses an anticancer agent comprising as the active ingredient docosahexaenoyl-monoglyceride. Japanese Patent Un-examined Publication No. 1-203331 discloses an anticancer agent comprising as the active ingredient 1,2-didocosahexaenoylphosphatidylcholine. Japanese Patent Un-examined Publication No. 3-163031discloses a liquid-film anticancer agent wherein an anticancer agent is added to mixed molecular aggregate of phospholipids and micellar-forming surfactants. Japanese Patent Un-examined Publication No. 2-11516 discloses an anticancer agent comprising one of phospholipids as the active ingredient. The anticancer mechanism of anticancer agents comprising known phospholipids remains completely unknown, and furthermore, there has been no research on anticancer activation due to apoptosis.

### Disclosure of the Invention

It is a first object of the present invention to provide a new apoptosis inducer comprising di(alkylcarbonyl) phosphatidylcholine or the like as the active ingredient.

It is a second object of the present invention to provide a new caspase cascade activator comprising di(alkylcarbonyl) phosphatidylcholine or the like as the active ingredient.

It is a third object of the present invention to provide a new anticancer agent having no side effects comprising di(alkylcarbonyl) phosphatidylcholine or the like as the active ingredient.

The present inventors focused on glycerophospholipids such as dimyristoyl phosphatidylcholine and the like, which are well known as a kind of phospholipid, and unexpectedly found that these compounds act as apoptosis inducers and caspase cascade activators, and obtained a new finding that these compounds can be used as highly-safe anticancer agents having no side effects in comparison with prior anticancer agent, whereby the present invention has been completed.

That is to say, the present invention provides an apoptosis inducer comprising, as the active ingredient, at least one member selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine.

The present invention also provides a caspase cascade activator comprising, as the active ingredient, at least one member selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine.

The present invention also provides an anticancer agent for bringing apoptosis of tumor cells by using the aforementioned apoptosis inducer or caspase cascade activator.

The present invention also provides an anticancer agent for bringing apoptosis of tumor cells by activating the caspase cascade using molecular aggregates comprising the active ingredients such as di(alkylcarbonyl) phosphatidylcholine or the like.

The present invention also provides an anticancer agent consisting of dimyristoyl phosphatidylcholine and pharmaceutically acceptable carriers and/or adjuvants.

### Brief Description of the Drawings

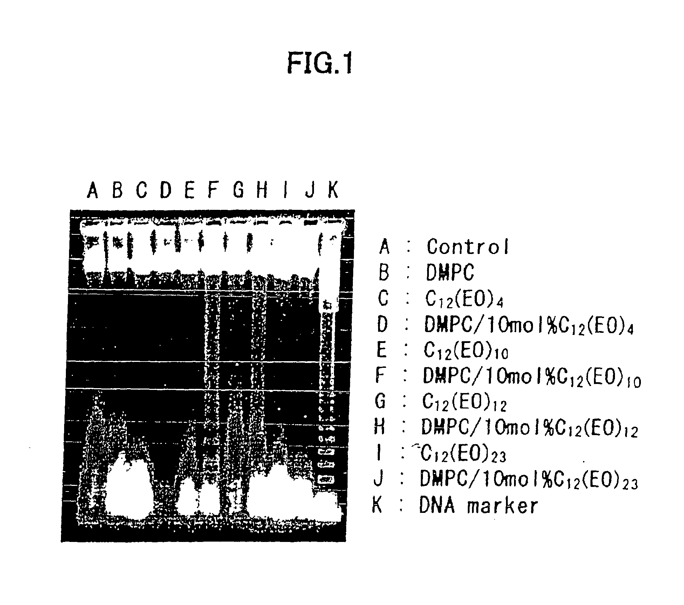

Fig. 1 illustrates agarose gel electrophoresis of DNA from HL-60 cells subjected to processing by hybrid liposomes.
   In the diagram, A indicates Control, B indicates DMPC, C indicates C₁₂(EO)₄, D indicates DMPC / 10 mol % C₁₂(EO)₄, E indicates C₁₂(EO)₁₀, F indicates DMPC / 10 mol % C₁₂(EO)₁₀, G indicates C₁₂(EO)₁₂, H indicates DMPC / 10 mol % C₁₂(EO)₁₂, I indicates C₁₂(EO)₂₃, J indicates DMPC / 10 mol % C₁₂(EO)₂₃, and K indicates a DNA marker;
Fig. 2 illustrates observation of HL-60 cells, which have been subjected to processing by hybrid liposome of DMPC / 10 mol % C₁₂(EO)₁₀, using a fluorescence microscope;
Fig. 3 is a diagram which illustrates caspase-9 inhibition effects by a liposome solution comprising dimyristoyl phosphatidylcholine;
Fig. 4 is a diagram which illustrates caspase-3 inhibition effects by a liposome solution comprising dimyristoyl phosphatidylcholine;
Fig. 5 is a diagram which illustrates caspase-1 / caspase-4 inhibition effects by a liposome solution comprising dimyristoyl phosphatidylcholine;
Fig. 6 is a diagram which illustrates detection of decomposed matter from caspase-3 substrates by the western blotting method. The drawing illustrates analysis of the western blotting method with regard to HL-60 cells subjected to processing with hybrid liposome comprising DMPC and C₁₂(EO)₁₀; and
Fig. 7 is a diagram which illustrates treatment experiment results for cancer bearing mice by the DMPC dispersion liquid. The drawing indicates a survival curve with regard to mice subjected to processing with the DMPC liposome (0 to 12 days) following B16-F0 melanoma cells being transplanted.

In the drawing, the solid circles (●) indicate control, the triangles (Δ) indicate [DPMC] = 5.0 × 10⁻² M, the squares (■) indicate [DPMC] = 5.0 × 10⁻³ M, and the diamonds (◇) indicate [DPMC] = 5.0 × 10⁻⁴ M.

### Best Mode for Carrying Out the Invention

In the present specification, "bring apoptosis" means effecting change characterized by drastic morphologic changes such as cell shrinkage, condensation of chromatin and fragmentation of DNA into nucleosome pieces (approximately 180 bp), which has been defined morphologically and biochemistrically in the field of medical science and biology, for performing programmed cell death, which is different from necrosis (local death). It is possible to confirm whether apoptosis of cells has occurred or not by a conventional method such as DNA ladder observation using electrophoresis and observation of morphology using an optical microscope.

In addition, in the present specification, "activate the caspase cascade" means effecting the state wherein, in a signal transmission network for apoptosis configured in cells by an Asp-specific-reactive cysteine protease (an enzyme which hydrolyzes the C-terminal of aspartate), which is referred to as caspase, some of caspases are activated, and accordingly, activation of all caspases is not required. The kinds of caspases which are activated may depend upon the kinds of cells, conditions, or the like.

The activation of the caspases can be estimated and confirmed using conventional methods such as a inhibition experiment with a flow cytometer using a caspase inhibitor, detection of decomposed portions from the caspases using a western blotting method, or the like.

In the present specification, "tumor cells" means cells, which constitutes so-called cancer and the like, or cells derived from or relating to the cancer cells, and are not restricted so long as these cells are cells which are called tumor cells in general. Furthermore, these cells include not only the cells situated in organic tissue as a disease, but also cultured cells, which are separated from homogenated tissue or are generally provided as cells for examination, hybridoma derived from the tumor cells and the like, and are also not restricted to human cells. Such tumor cells include cells constituting hematopoietic organ tumors such as acute leukemia, chronic leukemia, malignant lymphoma, multiple myeloma, macroglobulinaemia and the like, and cells constituting solid tumors such as brain tumors, head and neck cancer, mammary carcinoma, pulmonary carcinoma, esophagus cancer, stomach carcinoma, colon carcinoma, hepatic carcinoma, gallbladder cancer, cholangioma, pancreatic cancer, islet cell cancer, renal cell cancer, adrenocorrical carcinoma, bladder cancer, prostatic carcinoma, testis tumor, ovarian carcinoma, uterine carcinoma, chorionic carcinoma, thyroid cancer, malignant carcinoid tumor, cutaneous carcinoma, malignant melanoma, osteogenic sarcoma, soft tissue tumors, neuroblastoma, wilms tumors, embryonal rhabdomyosarcoma, retinoblastoma, and the like.

In the present specification, the term "pharmaceutically acceptable carrier or adjuvant" means a non-toxic carrier or adjuvant which can be administered along with dimyristoyl phosphatidylcholine to a patient and does not diminish the pharmaceutical activity of dimyristoyl phosphatidylcholine.

First of all, a first embodiment according to the invention will be described.

The first embodiment according to the present invention relates to an apoptosis inducer comprising, as the active ingredient, a compound selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine.

The apoptosis inducer of the present invention is effective in prevention or treatment for various types of diseases occurring due to the functions of normal apoptosis being inhibited. Such diseases include cell-multiplication diseases, deformities, autoimmune diseases and the like. In particular, the apoptosis inducer of the present invention brings apoptosis of tumor cells, which constitutes cancer and the like, wherein the mechanism of apoptosis directly or indirectly contributes, whereby the apoptosis inducer is effective in prevention or treatment for cell-multiplication diseases such as cancer.

Thus, the present invention also relates to a prophylactic agent or therapeutic agent for cell-multiplication diseases, deformities or autoimmune diseases, wherein the active ingredient is a compound selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine.

The present invention also relates to use of a compound selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine for preparing an apoptosis inducer.

The present invention also relates to a method for inducing apoptosis of tumor cells by brining a compound, as the active ingredient, selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine, into contact with said tumor cells.

The present invention is also relates to a preventing or treatment method for cell-multiplication diseases, deformities or autoimmune diseases, comprising the step of administering a compound, as the active ingredient, selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine, to a patient suffering from said diseases.

Two alkylcarbonyls contained in the active ingredients used in the present invention may be the same or different, but are preferably the same. The number of carbons in alkylcarbonyl groups is preferably 10 to 20, and is more preferably 10 to 18, and is even more preferably 12 to 16, and is further preferably 14. The alkyl moiety in the alkylcarbonyl group may be a straight chain or a branched chain.

When the active ingredient used in the present invention is di(alkylcarbonyl) phosphatidylalkanolamine, the number of carbon atoms of alkanol moiety is preferably 1 to 6, and is more preferably 1 to 3, and is further preferably 2.

The preferable active ingredient used in the present invention is represented by the following general formula (1):

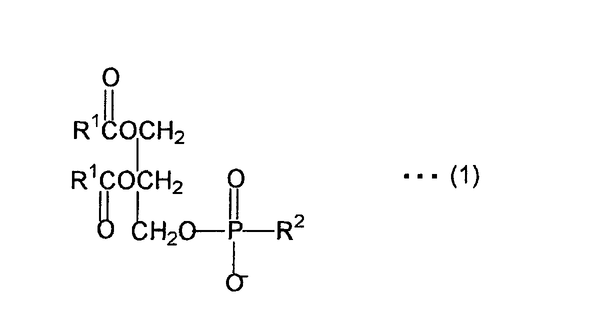

In the general formula (1), R¹ represents CH₃(CH₂)ₘ₋₂ wherein m is an integer of 10 to 18, and is preferably 12 to 16, and is more preferably 14.

R² is OCH₂CH₂N⁺(CH₃)₃, OCH₂CH₂NH₂ or OCH₂CH(NH₂)COOH, or a group represented by the following formula (2):

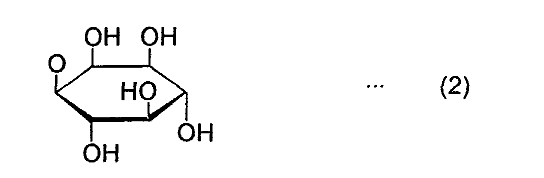

The active ingredient used in the present invention is preferably di(alkylcarbonyl) phosphatidylcholine.

The compound represented by the general formula (1) is most preferably dimyristoyl phosphatidylcholine (DMPC) represented by the following general formula (3):

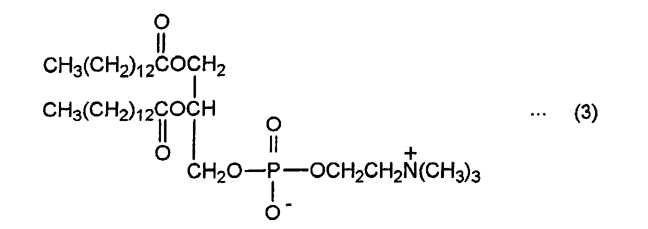

In this connection, dimyristoyl phosphatidylcholine is a kind of phospholipid which is referred to as phosphatidylcholine or lecithin, and is formally referred to as 1,2-dimyristoyl-sn-glycero-3-phosphocholine. Dimyristoyl phosphatidylcholine is sometimes referred to as dimyristoyllecithin or L-α- dimyristoyl phosphatidylcholine.

The active ingredient used in the invention may be used individually, or as a mixture of two or more compounds. When using the mixture of two or more compounds, the mixture of di(alkylcarbonyl) phosphatidylcholine, in particular, DMPC and other active ingredients are preferably employed.

The active ingredients such as di(alkylcarbonyl) phosphatidylcholine used in the present invention may originate from naturally occurring materials or chemically synthesized materials. When the active ingredients originate from naturally occurring materials, the materials can be obtained by extracting, separating or purifying from raw materials such as animals, plants, or microorganisms, e.g., brains, livers, yolks of eggs, soybeans, or yeast, with conventional methods. Also, the active ingredients can be obtained by using a conventional separating method which uses the difference in the length between the chains of fatty acids from commercially-available lecithin as a raw material. When the active ingredients originate from chemically synthesized materials, the active ingredients are easily synthesized by using knowledge of known synthetic chemistry. For example, in a case of dimyristoyl phosphatidylcholine, it can be obtained by a technique wherein myristic acid is introduced into glycerophosphocholine with a conventional method, or the like. Also, commercially-available products can be used, and highly-pure medical-grade products are preferably used. For example, DMPC is marketed under a product name of COATSOME MC-4040 by NOF Corporation.

With the apoptosis inducer of the present invention, the active ingredient can be mixed with a known and pharmaceutically acceptable carrier, excipient, disintegrator, corrigent, bulking agent, diluent, solution adjuvant or the like, so as to prepare pharmaceutical compositions in the form of a tablet, capsule, granules, powder, pill, liquid medicine, drinkable preparation, injection, infusion, suppository or the like, for example, according to conventional methods. These preparations may be administered either orally or parenterally.

When the apoptosis inducer of the present invention is used for treatment or the like of diseases, the dosage amount varies depending upon the administered subject, the administering route, the conditions of the patient or the like. When the apoptosis inducer is administered into the blood, it is preferable to administer a solution comprising the active ingredient with a concentration of 1 × 10⁻¹ to 1 × 10⁻⁶ M in a dosage amount of 0.01 to 100 ml/kg-body weight, about 1 to 3 times per day. Administration of a dosage amount of 1 to 50 ml/kg-body weight of a solution comprising the active ingredient with a concentration of 5 × 10⁻² to 5 × 10⁻⁴ M, about 1 to 3 times per day is more preferable.

Next, a second embodiment according to the present invention will be described.

The second embodiment according to the present invention relates to a caspase cascade activator comprising, as the active ingredient, a compound selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine.

The caspase cascade activator of the present invention is effective in prevention or treatment of various types of diseases occurring due to the functions of normal activation of the caspase cascade being inhibited. Such diseases include cell-multiplication diseases, deformities, autoimmune diseases and the like. In particular, the caspase cascade activator of the present invention prompts the apoptosis mechanism to be normalized by activating the caspase cascade, and thus is effective in prevention or treatment of cell-multiplication diseases such as tumor cells or the like, which constitutes cancer or the like.

Thus, the present invention also relates to use of a compound selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine for preparing a caspase cascade activator

The present invention also relates to a method for activating the caspase cascade of tumor cells by bringing a compound, as the active ingredient, selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine, into contact with said tumor cells.

The active ingredients used in the cascade activator are the same as those described regarding the apoptosis inducer.

With the caspase cascade activator of the present invention, the active ingredients can be mixed with a known and pharmaceutically acceptable carrier, excipient, disintegrator, corrigent, bulking agent, diluent, solution adjuvant, or the like, so as to prepare pharmaceutical compositions in the form of a tablet, capsule, granules, powder, pill, liquid medicine, drinkable preparation, injection, infusion, suppository or the like, for example, according to conventional methods. These preparations may be administered either orally or parenterally.

When the caspase cascade activator of the present invention is used for treatment or the like of diseases, the dosage amount varies depending upon the administered subject, the administering route, the conditions of the patient or the like. When the caspase cascade activator is administered by injection or infusion, it is preferable to administer a solution comprising the active ingredient with a concentration of 1 × 10⁻¹ to 1 × 10⁻⁶ M in a dosage amount of 0.01 to 100 ml/kg-body weight, about 1 to 3 times per day. Administration of a dosage amount of 1 to 50 ml/kg-body weight of a solution comprising the active ingredient with a concentration of 5 × 10⁻² to 5 × 10⁻⁴ M, about 1 to 3 times per day is more preferable.

Finally, a third embodiment according to the present invention will be described.

The third embodiment according to the present invention relates to an anticancer agent comprising at least one member, as the active ingredient, selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine. The active ingredients used in the anticancer agent of the invention are the same as those described regarding the apoptosis inducer.

The anticancer agent of the invention can activate the caspase cascade of the tumor cells or the like by effectively activating the tumor cells or the like, wherein normal functions of the caspase cascade have been inhibited, to prevent or treat cancer. The subject diseases for the anticancer agent of the invention is not restricted, so long as that it is so-called cancer. In particular, the anticancer agent of the invention is particularly excellent in prevention or treatment of cancer which is classified into hematopoietic organ tumors or solid tumors, in particular, such as acute leukemia, chronic leukemia, malignant melanoma, pulmonary carcinoma, hepatic carcinoma, stomach carcinoma, and malignant brain tumors.

Thus, the present invention also provides a prophylactic agent or therapeutic agent for acute leukemia, chronic leukemia, malignant melanoma, pulmonary carcinoma, hepatic carcinoma, stomach carcinoma and malignant brain tumor, wherein the active ingredient is a compound selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine.

The present invention also relates to use of a compound selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine for preparing an anticancer agent.

The present invention relates to a preventing or treatment method for any of acute leukemia, chronic leukemia, malignant melanoma, pulmonary carcinoma, hepatic carcinoma, stomach carcinoma or malignant brain tumor, comprising the step of administering a compound, as the active ingredient, selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine, to a patient suffering from said diseases

The anticancer agent of the invention can be prepared by mixing at least one member selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine with a known and pharmaceutically acceptable carrier, excipient, disintegrator, corrigent, bulking agent, diluent, solution adjuvant or the like, so as to obtain pharmaceutical compositions freely in the form of a tablet, capsule, granules, powder, pill, liquid medicine, drinkable preparation, injection, infusion, suppository or the like. These preparations can be administered either orally or parenterally. In particular, the anticancer agent is preferably administered as a drinkable preparation, injection or infusion.

When administers as an injection, infusion or drinkable preparation, the active ingredients such as di(alkylcarbonyl) phosphatidylcholine and the like are preferably contained in molecular assemblies so as to be administered. Examples of the molecular aggregates include fatty emulsifier, polymer micelle, complex with protein such as albumin, liposome and the like. Among them, liposome is most preferably used for the major reason that the active ingredient have functions for forming themselves into liposomes in an aqueous solvent.

When forms liposomes, the concentration of the active ingredient is preferably 5 × 10⁻⁴ to 1.0 × 10⁻¹ M, more preferably 1 × 10⁻² to 5 × 10⁻² M.

The liposome of the invention may be formed with any conventional method. For example, a conventional preparation method such as the thin film method, reversed phase evaporation method, freezing-melting method, ethanol injection method, high-pressure emulsion method, ultrasonic dispersion method, dialysis method, extrusion method, or the like, can be employed as appropriate. Furthermore, the method described in Japanese Patent Un-examined Publication No. 9-87168, etc., may be used.

As an aqueous solvent available to preparation of liposome, plain water can be used, or an aqueous mixture solvent comprising a small amount of ethanol, or also plasma, can be used, official water for injection, distilled water, ultrapure water, or the like is preferably used. A solution may be used wherein known physiologically active substances, proteins, buffer substances, various kinds of salt, plasma, saccharide, or the like are dissolved in an aqueous solvent.

The liposome thus obtained can be purified with a method such as ultrafiltration, centrifugal separation, gel filtration technique. Also, operations such as concentration, dilution or the like may be freely performed.

Also, the liposome of the invention may comprise known liposome-forming components to some degree not departing from the object of the present invention. Specifically, the components include phospholipids such as egg yolk lecithin, soybean lecithin, hydrogenated egg yolk lecithin, hydrogenated soybean lecithin, phosphatidyl-glycerol.

Also, cholesterol can be used as a stabilizer for liposome membrane in a small amount. Alarge amount of cholesterol might diminish anticancer activation of dimyristoyl phosphatidylcholine, and accordingly is not preferably used. Also, phosphatidic acid or fatty acid may be added as a charged substance.

Furthermore, alkyl-PEG having 10 to 20 carbon atoms, represented by polyoxyethylene dodecylether, or a surfactant known as commercially-available product name, "TRITON", "PLURONIC", or the like may be added as a dispersion stabilizer. Among them, alkyl-PEG having 12 to 14 carbon atoms is preferably added. In particular, hybrid liposome comprising di(alkylcarbonyl) phosphatidylcholine and a surfactant which may form micelle, is preferably used. Even more particularly, a combination of DMPC and alkyl-PEG having 10 to 16 carbon atoms and an EO number of 2 to 30 is preferable (preferably, alkyl-PEG having 12 to 14 carbon atoms and the EO number of 2 to 24).

Furthermore, with the present invention, saccharide is further preferably added into an aqueous solvent in order to contribute to dispersion stabilization for liposome, and enhance the anticancer activation. As available saccharide, known saccharide such as monosaccharide, disaccharide, oligosaccharide, polysaccharide, or the like, can be freely used according to the object without limitations as such, and in particular, sucrose, trehalose, or the like, is preferably used. Addition amounts thereof can be determined as appropriate. Furthermore, α-tocopherol or the like may be added into liposome as an antioxidant.

The diameter of the liposome prepared may be any length so long as the diameter is within pharmaceutically acceptable range, and is preferably about 20 nm to 1 µm, and is more preferably about 30 nm to 300 nm, and is further preferably about 40 nm to 180 nm, in order to enhance anticancer activation. The liposome is preferably prepared so as to uniformly disperse into an aqueous solvent. Occurrence of precipitation, aggregate or the like, leads to difficulty of usage as an anticancer agent mainly due to physical reasons, and accordingly is undesirable.

When the anticancer agent of the invention is used for treatment or the like of diseases, the dosage amount of the anticancer agent of the invention varies depending upon the subject of administration, the administration route, the conditions of the patient, or the like. When the anticancer agent is administered into the bloods, it is preferable to administer a solution comprising the active ingredient with a concentration of 1 × 10⁻¹ to 1 × 10⁻⁶ M in a dosage amount of 0.01 to 100 ml/kg-body weight, preferably, a solution comprising the active ingredient with a concentration of 5 × 10⁻² to 5 × 10⁻⁴ M in a dosage amount of 1 to 50 ml/kg-body weight, about 1 to 3 times per day.

### Examples

The present invention will be more specifically described with reference to examples, but the present invention is not intended to be restricted to the examples.

### Example 1

### <Apoptosis inducing experiment using liposome>

### 1-1. Preparation of liposome

In order to prepare a hybrid liposome solution, a predetermined quantity of dimyristoyl phosphatidylcholine (product name COATSOME MC-4040, manufactured by NOF Corporation) and polyoxyethylene-dodecyl-ether (commercially-available product, manufactured by Sigma Chemical Company) was weighed and placed in an eggplant type flask into which PBS had been poured. It was subjected to ultrasonic irradiation by a bath-type ultrasonic irradiator (product name, BRANSONIC MODEL B2210:90W) under conditions of 45°C and 1 ml/min, and the obtained solution was further filtered with a 0.45 µm filter (product name ADVANTEC DISMC-13cp).

### 1-2. Results of DNA ladder observation by electrophoresis, and cell fragmentation observation with a fluorescence microscope

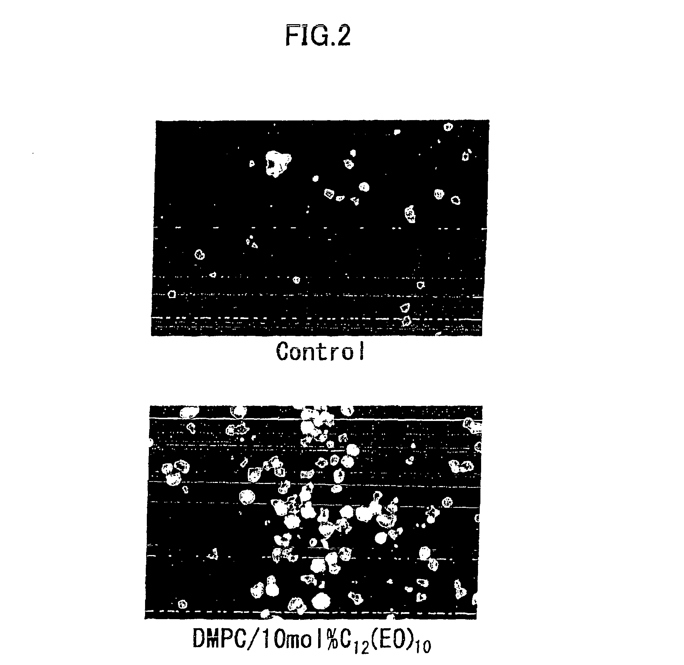

Human promyelocytes of leukemia (HL-60, purchased from Riken Cell Bank) were subjected to processing using the hybrid liposome prepared in section 1-1, and DNAladder observation was performed by agarose gel electrophoresis. Furthermore, cell fragmentation was observed with a fluorescence microscope using fragmentation DNA dyeing method. The results are shown in Fig. 1 and Fig. 2, respectively.

### Example 2

### <Caspase-9 inhibiting experiment using a flow cytometer>

### 1-1. Preparation of reagents

RNase (a commercially-available product, manufactured by Amresco) was dissolved into -PBS so as to prepare a RNase solution with a concentration of 0.25 mg/ml. The obtained solution was kept in a freezer until the experiment (prior to the experiment, the solution was restored to room temperature).

Propidium Iodite (a commercially-available product, manufactured by Funakoshi Co., Ltd., which will be abbreviated as PI) was dissolved into -PBS so as to prepare a PI solution with a concentration of 0.5 mg/ml. The obtained solution was shielded from light, and was kept in cold storage until the experiment (prior to the experiment, the solution was restored to room temperature).

99% ethanol (a commercially-available product, manufactured by Nacalai Tesque, Inc.) and -PBS were mixed at a ratio of 7:3 to prepare 70% ethanol. The obtained solution was kept in a freezer until the experiment (prior to the experiment, the solution was restored to room temperature).

11.0 mg of Caspase-9 inhibitor (Ac-L-Leu-L-Glu-L-His-L-Asp-H, a commercially-available product, manufactured by PEPTIDE INSTITUTE, INC, sometimes abbreviated as Ac-LEHD-CHO) was weighed and was dissolved into 404 µl of DMSO (dimethyl-sulfoxide, a commercially-available product). The obtained solution was kept in a freezer with the exterior of the vessel dry until the experiment (prior to the experiment, the solution was restored to room temperature).

In order to prepare a hybrid liposome solution, dimyristoyl phosphatidylcholine (product name, COATSOME MC-4040 produced by NOF Corporation, sometimes abbreviated as DMPC) and polyoxyethylene dodecylether (C₁₂(EO)₁₀, a commercially-available product, produced by Sigma Chemical Company) were weighed and placed in an eggplant type flask into which PBS had been poured, so as to form a solution with a concentration of dimyristoyl phosphatidylcholine of 1.5 × 10⁻² M, and with a concentration of polyoxyethylene dodecylether of 1.67 × 10⁻³ M. The solution was then subjected to ultrasonic irradiation by a bath-type ultrasonic irradiator (product name, BRANSONIC MODEL B2210:90W) under conditions of 45°C and 1 ml/min. The solution thus obtained was further filtered with a 0.45 µm filter (product name, ADVANTEC DISMC-13cp). With the liposome solution comprising dimyristoyl phosphatidylcholine thus obtained, it has been confirmed that the diameter of liposome is approximately 76 nm, as a result of particle size measurement with a dynamic light scattering method using electrophoretic light-scattering photometer (product name Photal ELS-8000, produced by OTUKA ELECTRONICS Corporation). Also, it was confirmed that the liposome was stable over 1 month from the particle size measurement with time.

### 1-2. Caspase inhibition experiment

The following experiment was performed using the reagents prepared in section 1-1. First of all, multiple samples were prepared in stages wherein 0 to 120 µl of caspase-9 inhibitor was added to 12 ml of a suspension (50 × 10⁴ cells/ml) of human promyelocytes of leukemia (HL-60, purchased from Riken Cell Bank). Then, the mixture were incubated for 90 minutes, 2 ml of liposome solution comprising dimyristoyl phosphatidylcholine was each added to the samples. Subsequently, each of the samples were incubated for three hours. After washing each sample with 2 ml of -PBS, 5 ml of 70% ethanol was added to each sample, and each sample was left at freezing temperature for one hour so as to be subjected to tissue-fixing. Furthermore, following washing each sample with 2 ml of -PSB, 6 ml of RNase solution was added to each sample, and subsequently, each sample was incubated for 30 minutes. Next, following adding 670 µl of PI solution, each sample was shielded from light with aluminum foil, and was left in a dark place at freezing temperature for 30 minutes, and was subjected to PI dyeing. After PI dyeing, each sample was subjected to centrifugal separation at 2000 rpm for five minutes. After removal of supernatant, and the precipitation formed was suspended in 500 µl of -PBS so as to measure DNA content using a flow cytometer (product name, COULTER EPICS-XL).

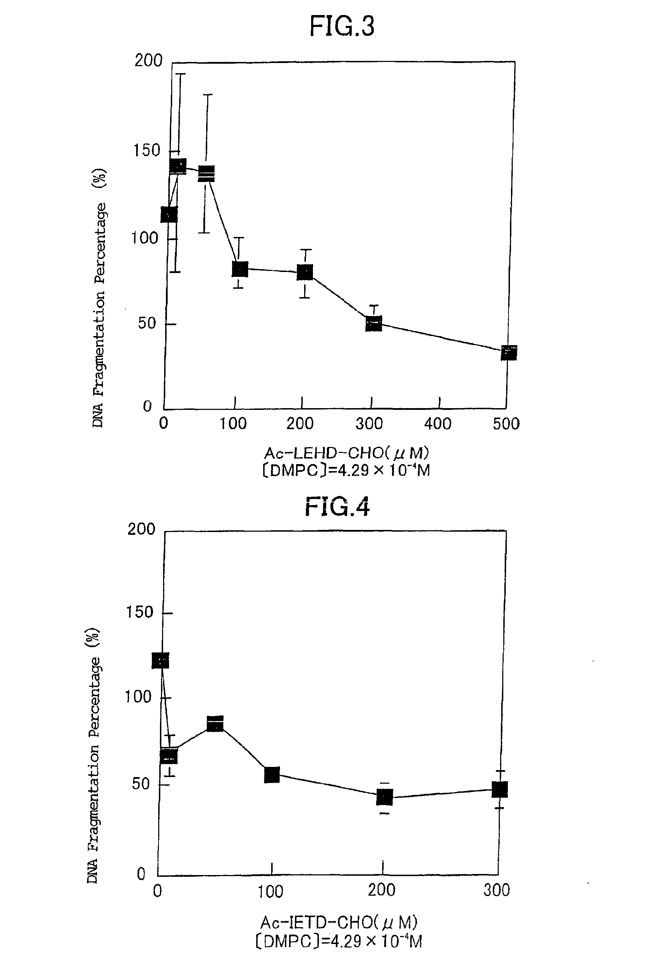

Inhibition effects were evaluated by the ratios of the DNA fragmentation percentage of the control (wherein no caspase-9 inhibitor was added, and only a liposome solution comprising dimyristoyl phosphatidylcholine was added) to the DNA fragmentation percentages of the samples wherein caspase-9 inhibitor was added in stages. The results are shown in Fig. 3.

### 1-3. Results

As is apparent from Fig. 3, it was observed that the DNA fragmentation percentage decreased approximately depending upon the concentration of the caspase-9 inhibitor. It suggests that activation of caspase-9 contributes to DNA fragmentation of human promyelocytes of leukemia in a liposome solution comprising dimyristoyl phosphatidylcholine.

With the control wherein the caspase-9 inhibitor had not been added, and only dimyristoyl phosphatidylcholine had been added, DNA fragmentation of human promyelocytes of leukemia was clearly observed, and it was confirmed that apoptosis was induced.

### Example 3

### <Procaspase-3 catabolic enzyme inhibition experiment using a flow cytometer>

### 2-1. Preparation of reagents

5.4 mg of procaspase-3 catabolic enzyme inhibitor (Ac-L-Ile-L-Glu-L-Thr-L-Asp-H, a commercially-available product produced by PEPTIDE INSTITUTE, INC and sometimes abbreviated as Ac-IETD-CHO) was weighed and dissolved into 355 µl of DMSO (dimethylsulfoxide, commercially-available product). The obtained solution was kept in a freezer with the exterior of the vessel dry until the experiment (prior to the experiment, the solution was restored to room temperature).

### 2-2. Caspase inhibition experiment

The experiment was performed in the same way as in section 1-2 of Example 1, except for using the procaspase-3 catabolic enzyme inhibitor prepared in section 2-1 instead of the caspase-9 inhibitor prepared in section 1-1 of Example 1.

### 2-3. Results

As is apparent from Fig. 4, it was observed that the DNA fragmentation percentage decreased approximately depending upon the concentration of the procaspase-3 catabolic enzyme inhibitor. It suggests that activation of caspase-3 contributes to DNA fragmentation of human promyelocytes of leukemia in a liposome solution comprising dimyristoyl phosphatidylcholine.

### Example 4

### <Caspase-1,4 inhibition experiment using a flow cytometer>

### 3-1. Preparation of reagents

5.7 mg of caspase-1,4 inhibitor (Ac-L-Tyr-L-Val-L-Ala-L-Asp-H, a commercially-available product produced by PEPTIDE INSTITUTE, INC, sometimes abbreviated as Ac-YVAD-CHO) was weighed and dissolved into 382 µl of DMSO (dimethyl-sulfoxide, a commercially-available product). The obtained solution was kept in a freezer with the exterior of the vessel dry until the experiment (prior to the experiment, the solution was restored to room temperature).

### 3-2. Caspase inhibition experiment

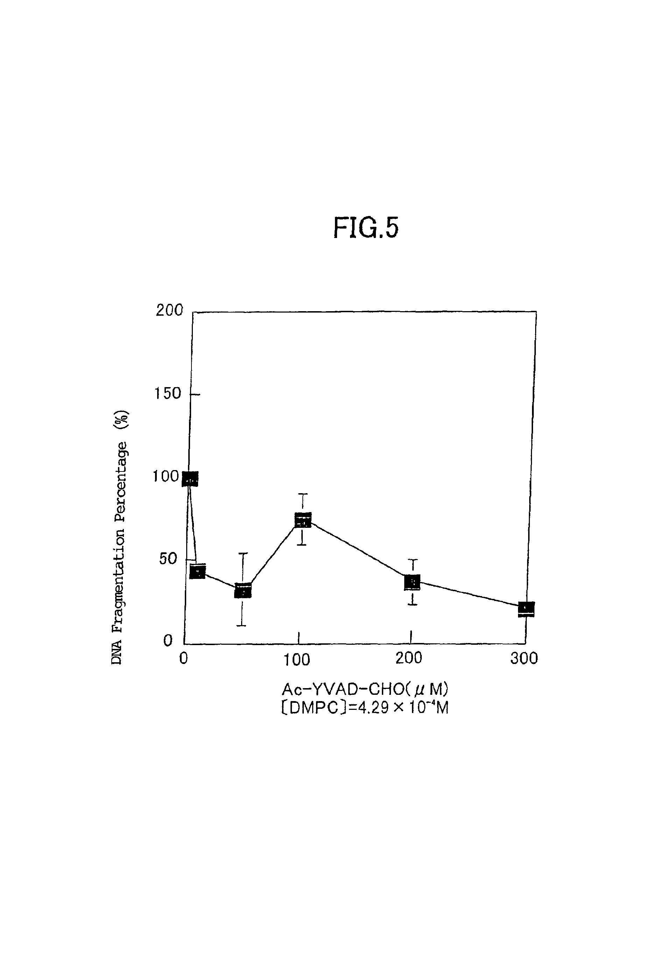

The experiment was performed in the same way as in section 1-2 of Example 1, except for using of the caspase-1,4 inhibitor prepared in section 3-1 instead of the caspase-9 inhibitor prepared in section 1-1 of Example 1. The results are shown in Fig.5.

### 3-3. Results

As is apparent from Fig. 5, it was observed that the DNA fragmentation percentage decreased approximately depending upon the concentration of the caspase-1,4 inhibitor. It suggests that activation of caspase-1 or caspase-4 contributes to DNA fragmentation of human promyelocytes of leukemia in a liposome solution comprising dimyristoyl phosphatidylcholine.

### Example 5

### <Detection of decomposed matter from substrates of caspase-3 with a western blotting method>

### 4-1. Description of detection of decomposed matter from substrates of caspase-3 with a western blotting method

Upon adding SDS, an anionic surfactant, into a protein sample wherein disulphide bonds have been cut off using a reducing agent such DTT or the like, the protein is denatured, and the SDS is quantitatively connected to the protein, and thus the protein can be separated according to the molecular weight using electrophoresis. The separated protein is electrically blotted onto a nitrocellulose membrane. Then, a primary antibody (Anti-PARP p85 Fragment rabbit polyclonal antibody), which is specifically connected to decomposed matter of one of substrates of caspase-3, poly(ADP-ribose) polymerase (which will be abbreviated as PARP), is connected thereto. Subsequently, secondary antibody (Donkey Anti-Rabbit IgG(H+L) Alkaline Phosphatase conjugate) labeled with alkaline-phosphatase is further connected, followed by addition of BCIP and NBT, which are substrates of alkaline-phosphatase. As a result, insoluble purple precipitation occurs due to cutting off of phosphoric acids of BCIP and reduction of NBT, whereby the antibody can be detected.

### 4-2. List of reagents for detecting decomposed matter from substrates using a western blotting method

- Sample Buffer (1.0 M Tris-HCl (pH 6.8), 40% SDS, 25% Glycerol, 1% Bromophenol Blue, 0.13 M DTT)
- Trans Buffer (0.025 M Tris, 0.2 M Glycine, 10% methanol)
- TBST (0.02 M Tris, 10% Tween20, 0.05 M, NaCl)
- Blocking Buffer (TBST + 5% skim milk)
- Anti-PARP p85 Fragment pAb (produced by Promega Corporation)
- Donkey Anti-Rabbit IgG (H+L)AP (produced by Promega Corporation)
- Tween20 (produced by Promega Corporation)
- Glycine (produced by Ameresco, Inc.)
- High-grade methanol (produced by Nacalai Tesque, Inc.)
- Skim milk (produced by Snow Brand Milk Products Co., Ltd.)
- Sodium Lauryl Sulfate (produced by Nacalai Tesque, Inc.)
- Blot Qualified BSA (produced by Promega Corporation)
- Tris(hydroxymethyl) aminomethane (produced by Nacalai Tesque, Inc., abbreviated as Tris)
- Bromophenol Blue (produced by Wako Pure Chemical Industries, Ltd.)
- Hydrochloric Acid (produced by Nacalai Tesque, Inc.)
- Glycerol (a GIBCO BRL product)
- Dithiothreitol (produced by Nacalai Tesque, Inc., abbreviated as DTT)
- Migration Buffer (produced by Bio-Rad Laboratories, Inc., 10 × Tris / Glycine /SDS/ buffer for SDS-PAGE applications)
- CBB R-250 dyeing liquid (produced by Bio-Rad Laboratories, Inc.)
- CBB R-250 bleach liquid (produced by Bio-Rad Laboratories, Inc.)
- Kaleidoscope prestained standards (produced by Promega Corporation)
- READY-GELS J for Polyacrylamide Electrophoresis (produced by Bio-Rad Laboratories, Inc.)
- Western Blue Stabilized Substrate for Alkaline Phosphatase (produced by Promega Corporation)

### 4-3. Operations of detection experiment for discomposed matter from caspase-3 substrate using western blotting method

The following experiment was performed using the reagents described in section 4-2. First of all, 2 ml of liposome solution comprising dimyristoyl phosphatidylcholine ([DMPC] = 1.5 × 10⁻² M, [C₁₂(EO)₁₀] = 1.67 × 10⁻³ M) was added to 12 ml of suspension (50 × 10⁴ cells/ml) of human promyelocytes of leukemia (HL-60, purchased from Riken Cell Bank). Then, the mixture was incubated for three hours. After washing the sample with 2 ml of -PBS, the mixture was subjected to centrifugal separation at 2000 rpm for six minutes. After removal of supernatant, -PBS (400 µl) and 5 × Sample Buffer (100 µl) was added. Subsequently, human promyelocytes of leukemia were crushed by a POLYTRON (product name, POLYTRON PT3100, produced by KINEMATICAAG, INC.) emulsifier (at 15000 rpm for 5 minutes), and furthermore, were subjected to ultrasonic irradiation by a bath-type ultrasonic irradiator under conditions of 37°C and 10 minutes, whereby homogenates were formed. Subsequently, the sample was warmed by a hot water bath at 100°C for 5 minutes, and was subjected to centrifugal separation at 15000 rpm for 30 minutes. 15 µl of the supernatant was each poured into wells. After protein was separated using SDS-PAGE (200 V, 45 minutes), the protein was subjected to blotting (50 V, 50 min) onto a nitrocellulose membrane to block at 37°C for 30 minutes using the Blocking Buffer. The primary antibody was added onto the transferred side thereof. After the reaction at room temperature for 90 minutes, the sample was washed with TBST buffer. Subsequently, the secondary antibody was added onto the side where the primary antibody had been connected. After the reaction at room temperature for 90 minutes, the sample was washed with TBST buffer. Further washing with demineralized water, chromogenic substrates was added so as to detect PARP decomposed matter. The results are shown in Fig. 4.

### 4-4. Results of detection experiment of caspase-3 substrate decomposed matter

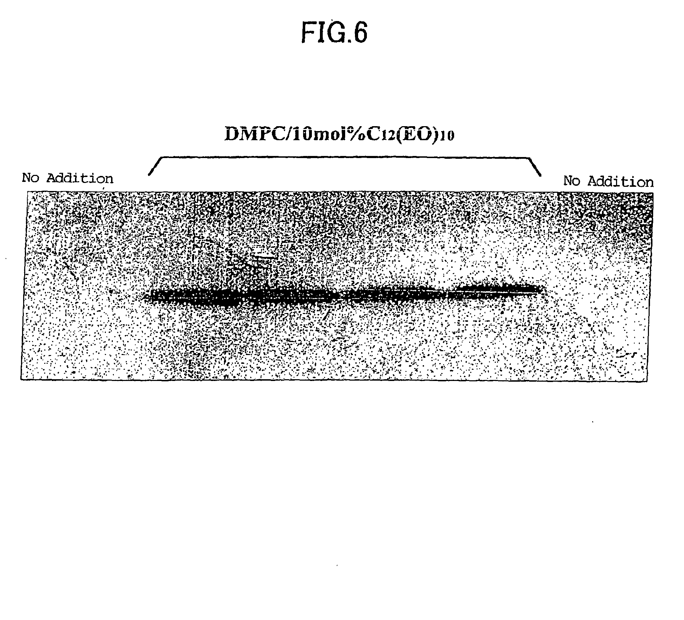

Caspase-3 is activated at a terminal of the caspase cascade activation in apoptosis signals, and decomposes a great number of substrates to induce apoptosis. In the experiment in section 4-3, decomposed matter from PARP, one of caspase-3 substrates, was detected with a western blotting method. As a result, activation of caspase-3 within human promyelocytes of leukemia could be estimated. As is apparent from Fig. 6, when the sample was subjected to processing in a liposome solution comprising dimyristoyl phosphatidylcholine, PARP decomposed matter was detected. However, when the sample was subjected to processing in a liposome solution without dimyristoyl phosphatidylcholine, PARP decomposed matter was not detected. Accordingly, it suggests that caspase-3 is activated within human promyelocytes of leukemia by a liposome solution comprising dimyristoyl phosphatidylcholine.

### Example 6

### <Antitumor experiment for melanoma using DMPC>

### 5-1. Preparation of DMPC dispersion liquid

A predetermined quantity of DMPC (product name COATSOME MC-4040, a commercially-available, product produced by NOF Corporation) was weighed. -PBS was added as an aqueous buffer solution, and then subjected to ultrasonic irradiation under conditions of 45°C and 1 ml/min by a bath-type ultrasonic irradiator (product name, BRANSONIC MODEL B2210:90W). The obtained solution was filtered with a 0.45 µm filter (product name, ADVANTEC DISMC-13cp) to prepare DMPC dispersion liquid. In this connection, three DMPC solutions were prepared with concentrations of 5.0 × 10⁻² M, 5.0 × 10⁻³ M, and 5.0 × 10⁻⁴ M, by alternating the weight of DMPC. 5-2. In vivo treatment experiment using cancer bearing mice

For the animals, C57BL/6 female mice (aged five weeks, purchased from Charles River Japan) were quarantined and acclimated for five days, and used for experimentation from the age of six weeks in a group of six. The confinement environment was that of a temperature of 24 ± 2°C and a humidity of 55 ± 10%, and ingestion of water and food was unrestricted.

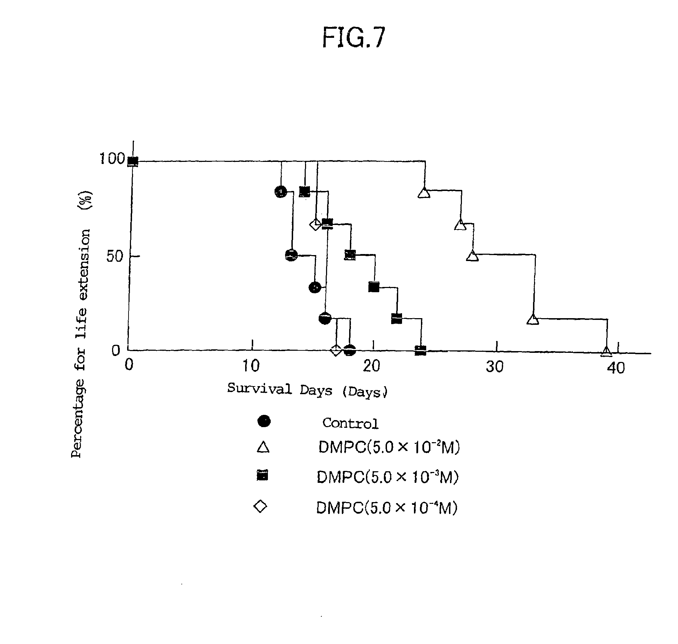

The mice were grouped six per group by stratified randomization based on the body weight at the day of transplanting cancer cells. B16-F0 melanoma cells (5.0 × 10⁵ cells) were transplanted into the abdominal cavities of the mice, thereby creating cancer bearing mice. The method of administering the DMPC dispersion liquid (prepared in section 5-1) was intraperitoneal administration, and the experiment was performed under an experiment schedule of a total of thirteen doses (20 ml / kg-body weight) of from the date of translplanting (one hour after translplating) to the twelfth day. After completion of administration, observation was carried out until all animals died, and the survival days of the mice was counted. Then the percentage for life extension was calculated by dividing the average survival days of the treated group by the average survival days of an untreated group and by multiplying a value thus obtained by 100. The results are shown in Fig. 7, and the effects of treatment were evaluated.

From the above results, it has been revealed that the DMPC dispersion liquid has remarkable treatment effects on cancer bearing mice. There has been no report so far of a DMPC single-component dispersion liquid having such cancer treatment effects, surprisingly. Accordingly, it has been newly discovered for the first time that DMPC, one of phospholipids, has high cancer-treating effects, and it has been found that DMPC can be expected to serve as a new cancer therapeutic agent with absolutely no side-effects.

The apoptosis inducer of the invention is capable of inducing apoptosis in tumor cells where the mechanism of apoptosis does not normally function under normal conditions. Also, the caspase cascade activator of the invention can activate the caspase cascade by inducing apoptosis in tumor cells wherein the mechanism of apoptosis does not normally function under normal conditions and accordingly the caspase cascade is not activated either, thereby carrying out apoptosis.

Further, the anticancer agent of the invention is extremely effective as a safe anticancer agent with no side-effects.

## Claims

1. An apoptosis inducer comprising, as the active ingredient, a compound selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine.

2. A caspase cascade activator comprising, as the active ingredient, a compound selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine.

3. The apoptosis inducer according to claim 1, wherein the alkylcarbonyl moiety has 10 to 20 carbon atoms and the alkanolamine moiety has 1 to 6 carbon atoms.

4. The caspase cascade activator according to claim 2, wherein the alkylcarbonyl moiety has 10 to 20 carbon atoms and the alkanolamine moiety has 1 to 6 carbon atoms.

5. The apoptosis inducer according to claim 1, wherein the active ingredient is dimyristoyl phosphatidylcholine.

6. The caspase cascade activator according to claim 2, wherein the active ingredient is dimyristoyl phosphatidylcholine.

7. An anticancer agent for bringing apoptosis of tumor cells by using the apoptosis inducer according to claims 1, 3 or 5.

8. An anticancer agent for bringing apoptosis of tumor cells by activating the caspase cascade using the caspase cascade activator according to claims 2, 4 or 6.

9. An anticancer agent for bringing apoptosis of tumor cells by activating the caspase cascade of said tumor cells using a molecular aggregate which comprises compounds selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine.

10. An anticancer agent according to claim 9, wherein said molecular aggregate is liposome.

11. An anticancer agent consisting of dimyristoyl phosphatidylcholine and pharmaceutically acceptable carriers and/or adjuvants.

12. A prophylactic agent or therapeutic agent for cell-multiplication diseases, deformities or autoimmune diseases, wherein the active ingredient is a compound selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine.

13. Aprophylactic agent or therapeutic agent for acute leukemia, chronic leukemia, malignant melanoma, pulmonary carcinoma, hepatic carcinoma, stomach carcinoma and malignant brain tumor, wherein the active ingredient is a compound selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine.

14. Use of a compound selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine for preparing an apoptosis inducer.

15. Use of a compound selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine for preparing a caspase cascade activator.

16. Use of a compound selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine for preparing an anticancer agent.

17. A method for inducing apoptosis of tumor cells by brining a compound, as the active ingredient, selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine, into contact with said tumor cells.

18. A method for activating the caspase cascade of tumor cells by bringing a compound, as the active ingredient, selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine, into contact with said tumor cells.

19. A preventing or treatment method for cell-multiplication diseases, deformities or autoimmune diseases, comprising the step of administering a compound, as the active ingredient, selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine, to a patient suffering from said diseases.

20. A preventing or treatment method for any of acute leukemia, chronic leukemia, malignant melanoma, pulmonary carcinoma, hepatic carcinoma, stomach carcinoma or malignant brain tumor, comprising the step of administering a compound, as the active ingredient, selected from the group consisting of di(alkylcarbonyl) phosphatidylcholine, di(alkylcarbonyl) phosphatidylalkanolamine, di(alkylcarbonyl) phosphatidylinositol and di(alkylcarbonyl) phosphatidylserine, to a patient suffering from said diseases.
